# EUROPEAN PATENT SPECIFICATION

(11) **EP 0 561 882 B1**
(45) Date of publication and mention of the grant of the patent: **31.08.1994**
(21) Application number: 92900468.7
(22) Date of filing: 08.10.1991
(51) Int. Cl.: C07C 275/64, C07C 259/06, C07C 273/18, A61K 31/17, A61K 31/16

(54) **HYDROXAMIC ACID DERIVATIVES WHICH INHIBIT LIPOXYGENASE**
DERIVATE VON HYDROXAMINSÄURE DIE LIPOXYGENASE HEMMEN
DERIVES D'ACIDE HYDROXAMIQUE INHIBANT LA LIPOXYGENASE

(30) Priority: 11.12.1990 JP 415745/90
(43) Date of publication of application: 29.09.1993
(73) Proprietor: PFIZER INC., Groton, CT 06340 (US)
(72) Inventor: ANDO, Kazuo, Chita-gun, Aichi 470-23 (JP); IKEDA, Takafumi, Handa-shi, Aichi (JP); NAKANE, Masami, Nagoya-shi, Aichi (JP)
(74) Representative: Wood, David John
(86) International application number: US9107193
(87) International publication number: WO9210469

(56) References cited:
- EP-A- 0 351 214
- EP-A- 0 384 594
- WO-A-90/12008

## Description

This invention relates to novel hydroxamic acid derivatives. The compounds of the present invention inhibit the enzyme lipoxygenase, and are useful in the treatment or alleviation of inflammatory diseases, allergy and cardiovascular diseases in mammals. This invention also relates to pharmaceutical compositions comprising such compounds and to the use of such compounds in treating inflammatory diseases, allergy and cardiovascular diseases in mammals. This invention further relates to methods of making such compounds.

Arachidonic acid is known to be the biological precursor of several groups of endogenous metabolites, prostaglandins including prostacyclins, thromboxanes and leukotrienes. The first step of arachidonic acid metabolism is the release of arachidonic acid and related unsaturated fatty acids from membrane phospholipids, via the action of phospholipase. Free fatty acids are then metabolized either by cyclooxygenase to produce the prostaglandins and thromboxanes or by lipoxygenase to generate hydroperoxy fatty acids which may be further converted to the leukotrienes. Leukotrienes have been implicated in the pathophysiology of inflammatory diseases, including rheumatoid arthritis, gout, asthma, ischemia reperfusion injury, psoriasis and inflammatory bowel disease. Any drug that inhibits lipoxygenase is expected to provide significant new therapy for both acute and chronic inflammatory conditions.

Recently several review articles on lipoxygenase inhibitors have been reported (See H. Masamune et al., Ann. Rep. Med. Chem., **24**, 71-80 (1989) and B. J. Fitzsimmons et al., Leukotrienes and Lipoxygenases, 427-502 (1989).

Compounds of the same general class as the compounds of the present invention are disclosed in EP 279263 A2, EP 196184 A2, JP 63502179 and U.S. patent No. 4,822,809.

The present invention provides novel hydroxamic acid derivatives of the formula 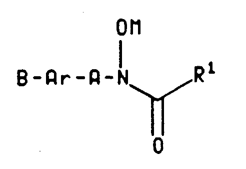
wherein R¹ is hydrogen, C1 to C4 alkyl, C2 to C4 alkenyl, alkylthioalkyl, alkoxyalkyl or NR²R³; R² and R³ are each independently hydrogen, C1 to C4 alkyl, hydroxy, aryl, or aryl substituted with one or more substituents selected from the group consisting of halo, nitro, cyano, C1 to C12 alkyl, C1 to C12 alkoxy, C1 to C12 halosubstituted alkyl, C1 to C12 hydroxysubstituted alkyl, C1 to C12 alkoxycarbonyl, aminocarbonyl, C1 to C12 alkylaminocarbonyl, di C1 to C12 alkylaminocarbonyl and C1 to C12 alkylsulfonyl, with the proviso that R² and R³ are not both hydroxy; M is hydrogen, a pharmaceutically acceptable cation, aroyl or C1 to C12 alkoyl; A is alkynylene or alkynylene substituted with one or more substituents selected from the group consisting of C1 to C4 alkyl, C2 to C4 alkenyl, cyano, halo, C1 to C4 alkoxy, C1 to C4 halosubstituted alkyl and C1 to C4 hydroxysubstituted alkyl; Ar is phenyl or naphthyl; B is hydrogen, C1 to C12 alkyl, C2 to C12 alkenyl, C1 to C12 halosubstituted alkyl, C1 to C12 hydroxysubstituted alkyl, OR⁴, NR⁴R⁵, aryl or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl; R⁴ and R⁵ are each independently hydrogen, C1 to C12 alkyl, C3 to C12 alkenyl, C3 to C8 cycloalkyl, aryl or 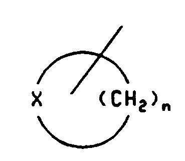
n is 4 to 6; X is O, NR⁶ or S; and R⁶ is hydrogen or C1 to C4 alkyl.

This invention also concerns pharmaceutical compositions comprising a pharmaceutically acceptable carrier or diluent and a compound of the invention or a pharmaceutically acceptable salt thereof.

### Definitions

As used herein, the following definitions are used.

"Halo" means radicals derived from the elements fluorine, chlorine, bromine and iodine.

"Alkyl" means straight or branched saturated hydrocarbon radicals, for example, methyl, ethyl, n-propyl and isopropyl.

"Alkenyl" means straight or branched unsaturated (double bonded) hydrocarbon radicals, for example, ethenyl, 1- or 2-propenyl, 2-methyl-1-propenyl and 1- or 2-butenyl.

"Alkynylene" means straight or branched unsaturated (triple bonded) hydrocarbon radicals, for example, -C≡C-, C≡CCH₂-, -C≡CHCH₂CH₂- and -C≡CCH(CN₃)-.

"Cycloalkyl" means carbocyclic radicals, for example, cyclopropyl, cyclobutyl, cyclopentyl and cyclohexyl.

"Alkoxy" means -OR⁷ wherein R⁷ is an alkyl radical, for example, methoxy, ethoxy, propoxy, isopropoxy and butoxy.

"Alkoxyalkyl" means -R⁸OR⁹ wherein R⁸ and R⁹ are independently alkyl radicals, for example, methoxymethyl, methoxyethyl, ethoxymethyl and ethoxyethyl.

"Alkylthioalkyl" means -R¹⁰SR¹¹ wherein R¹⁰ and R¹¹ are independently alkyl radicals, for example, methylthiomethyl, ethylthioethyl and methythioethyl.

"Alkoyl" means -COR¹² wherein R¹² is an alkyl radical, for example, formyl, acetyl, propionyl, butyryl and isobutyryl.

"Aryl" means aromatic radicals, for example, phenyl and naphthyl.

"Aroyl" means -COR¹³ wherein R¹³ is an aryl radical, for example, benzoyl and naphthoyl.

"Aryloxy" means -OR¹⁴ wherein R¹⁴ is an aryl radical, for example, phenoxy and naphthoxy.

"Alkoxycarbonyl" means -C(=0)R¹⁵ wherein R¹⁵ is an alkoxy radical, for example, methoxycarbonyl, ethoxycarbonyl and propoxycarbonyl.

"Alkylaminocarbonyl" means -C(=O)NHR¹⁶ wherein R¹⁶ is an alkyl radical, for example, methylaminocarbonyl, ethylaminocarbonyl and propylaminocarbonyl.

"Dialkylaminocarbonyl" means -C(=O)N(R¹⁷)R¹⁸ wherein R¹⁷ and R¹⁸ are independently alkyl radicals, for example, dimethylaminocarbonyl, diethylaminocarbonyl and methylethylaminocarbonyl.

"Alkylsulfonyl" means -SO₂R¹⁹ wherein R¹⁹ is an alkyl radical, for example, methanesulfonyl (mesyl) and ethanesulfonyl.

"Halosubstituted alkyl" means an alkyl radical as described above substituted with one or more halogens, for example, chloromethyl, trifluoromethyl and 2,2,2-trichloroethyl.

"Hydroxysubstituted alkyl" means an alkyl radical as described above substituted with one or more hydroxy radicals, for example, hydroxymethyl, dihydroxyethyl and trihydroxypropyl.

"Pharmaceutically acceptable cation" means non-toxic cations based on alkali and alkaline earth metals, for example, sodium, lithium, potassium, calcium and magnesium, as well as non-toxic ammonium, quaternary ammonium, and amine cations, for example, ammonium, tetramethylammonium, methylamine, dimethylamine, trimethylamine, ethylamine, diethylamine and triethylamine.

### Methods of Preparation

The compounds of the invention may be prepared by a number of synthetic methods. As used in the following reaction schemes, A, B and Ar are as defined above. Although in Schemes 1 and 2 R¹ is methyl and NH₂, respectively, compounds wherein R¹ is otherwise, as defined above, may be prepared in a similar manner.

In one embodiment, compounds of the formula (III) are prepared according to the reaction steps outlined in Scheme 1. 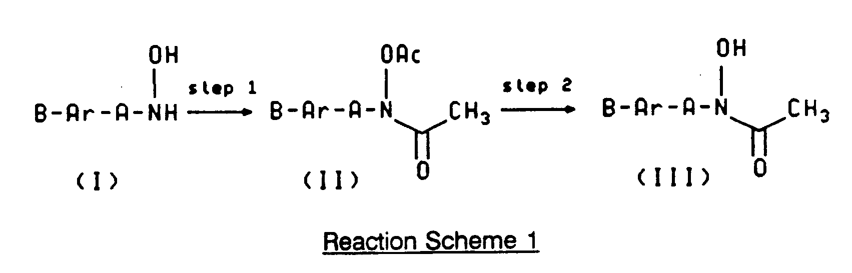
In step 1, the diacetyl compound (II) is prepared by standard methods known in the art. For example, the hydroxylamine (I) is reacted with acetyl chloride or acetic anhydride in a reaction-inert solvent in the presence of a suitable base. Preferred bases are sodium hydride, triethylamine and pyridine, with the latter two being particularly preferred. Suitable reaction-inert solvents include methylene chloride, chloroform, tetrahydrofuran, benzene and toluene. The reaction is usually carried out in the temperature range of about 0°C through to ambient temperature, with reaction times from 30 minutes to a few hours being typical. The product can be isolated and purified by conventional procedures, such as recrystallization or chromatography.

Step 2 involves selective hydrolysis of the diacetyl (II) with an appropriate base. Typical bases include ammonium hydroxide, sodium hydroxide, potassium hydroxide and lithium hydroxide preferably in methanol, ethanol, isopropyl alcohol or water, though binary solvent systems such as alcohol-water, tetrahydrofuran-water and the like may also be employed. Reaction temperatures are usually in the range of about -10°C through to ambient temperature, with the reaction usually complete within a few minutes to several hours. The product of formula (III) is isolated by standard methods and purification can be achieved by conventional means, such as recrystallization and chromatography.

In another embodiment, compounds of the formula (IV) are prepared as illustrated in Scheme 2. 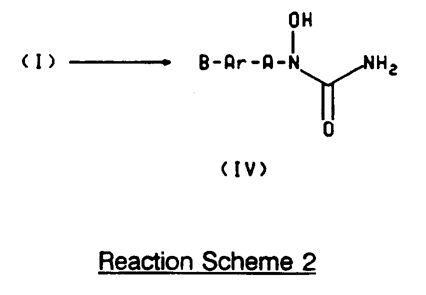
In this step the hydroxylamine (I) is treated with trimethylsilyl isocyanate in a reaction-inert solvent usually at ambient through to reflux temperature. Suitable solvents which do not react with reactants and/or products include, for example, tetrahydrofuran, dioxane, methylene chloride and benzene. An alternative procedure employs treatment of (I) with gaseous hydrogen chloride in a reaction-inert solvent such as benzene or toluene followed by treatment with phosgene. Reaction temperatures are usually in the range of ambient temperature through to boiling point of solvent. The intermediate carbamoyl chloride is not isolated but is subjected to (i.e. *in situ*) reaction with aqueous ammonia. The product of formula (IV) thus obtained is isolated by standard methods and purification can be achieved by conventional means, such as recrystallization and chromatography.

The aforementioned hydroxylamine (I) is easily prepared by standard synthetic procedures from readily available aldehyde. For example, the aldehyde is converted to its acetylene alcohol (see E.J. Corey et al., Tetrahedron Lett., 3769-3772 (1972)) and then the alcohol is treated with N,O-bis(*tert*-butoxycarbonyl)hydroxylamine under Mitsunobu-type reaction conditions followed by acid catalyzed hydrolysis of the N,O-protected intermediate product (see JP 1045344) to give the requisite hydroxylamine (I). N,O-diacetyl compound (II) can be prepared employing N,O-diacetyl hydroxylamine in place of N,O-bis(*tert*-butoxycarbonyl)hydroxylamine, thus providing a convenient route to the product of formula (III).

Alternatively, the hydroxylamine (I) can be prepared by direct coupling of the corresponding aryl bromide or aryl iodide and N,O-bis(*tert*-butoxycarbonyl)alkynylenehydroxylamine in the presence of (Ph₃P)₂PdCl₂ in a reaction-inert solvent such as diethylamine or triethylamine, followed by acid-catalyzed hydrolysis of the N,O-protected intermediate product to afford the desired hydroxylamine (I).

The hydroxylamine of formula (I) thus obtained by the aforementioned representative procedures is isolated by standard methods and purification can be achieved by conventional means, such as recrystallization and chromatography.

The pharmaceutically acceptable salts of the novel compounds of the present invention are readily prepared by contacting said compounds with a stoichiometric amount of, in the case of a non-toxic cation, an appropriate metal hydroxide or alkoxide or amine in either aqueous solution or a suitable organic solvent. In the case of non-toxic acid salt, an appropriate mineral or organic acid in either aqueous solution or a suitable organic solvent can be used. The salt may then be obtained by precipitation or by evaporation of the solvent.

### Biological Activity

The compounds of this invention inhibit lipoxygenase. This inhibition has been demonstrated by an assay using rat peritoneal cavity resident cells which determines the effect of such compounds on the metabolism of arachidonic acid.

The compounds of the examples were tested according to the methods described in "Synthesis of leukotrienes by peritoneal macrophages", Jap. J. Inflammation, **7**, 145-150 (1987), and were shown to be lipoxygenase inhibitors. In this test some preferred compounds exhibit low IC₅₀ values, in the range of about 0.01 to about 30µM, for lipoxygenase inhibition.

The ability of the compounds of the present invention to inhibit lipoxygenase makes them useful for controlling the symptoms induced by the endogenous metabolites arising from arachidonic acid in a mammalian subject. The compounds are therefore valuable in the prevention and treatment of such disease states in which the accumulation of arachidonic acid metabolites is the causative factor, e.g., allergic bronchial asthma, skin disorders, rheumatoid arthritis, osteoarthritis and thrombosis.

The compounds of the formula and their pharmaceutically acceptable salts are of particular use in the prevention and treatment of inflammatory diseases, allergy and cardiovascular diseases in a human subject.

### Methods of Administration

For treatment of the various conditions described above, the compounds of the invention and their pharmaceutically acceptable salts can be administered to a human subject either alone or, preferably, in combination with pharmaceutically acceptable carriers or diluents in a pharmaceutical composition, according to standard pharmaceutical practice. A compound can be administered via a variety of conventional routes of administration including orally, parenterally and by inhalation. When the compounds are administered orally, the dose range will generally be from about 0.1 to about 20 mg/kg/day, based on the body weight of the subject to be treated, preferably from about 0.1 to about 1.0 mg/kg/day in single or divided doses. If parenteral administration is desired, then an effective dose will generally be from about 0.1 to about 1.0 mg/kg/day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically acceptable salts can be administered, for example, in the form of tablets, powders, lozenges, syrups or capsules, or as an aqueous solution or suspension. In the case of tablets for oral use, carriers which are commonly used include lactose and corn starch. Lubricating agents, such as magnesium stearate, are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added.

For intramuscular, intraperitoneal, subcutaneous and intravenous use, a sterile solution of the active ingredient is usually prepared, and the pH of the solutions should be suitably adjusted and buffered. For intravenous use, the total concentration of solute should be controlled to make the preparation isotonic.

### Examples

The present invention is illustrated by the following examples. However, it should be understood that the invention is not limited to specific details of these examples. Proton nuclear magnetic resonance (NMR) spectra were measured at 270 MHz unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad.

### Example 1 N-Hydroxy-N-[4-(3-phenoxyphenyl)-3-butyn-2-yl]acetamide

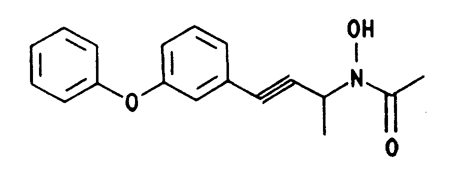

### Step 1, 3-phenoxyphenylethylene-1,1-dibromide

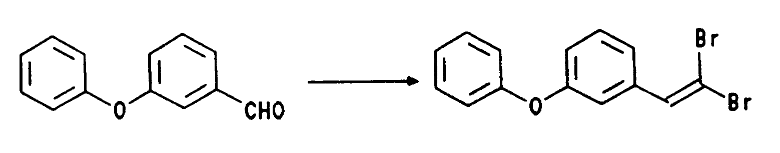

To a mixture of zinc (2.90 g, 0.044 mol), triphenylphosphine (Ph₃P, 11.6 g, 0.044 mol) in CH₂Cl₂ (100 ml) was added CBr₄ (14.7 g, 0.044 mol) at 0°C. After stirring for 30 minutes, 3-phenoxybenzaldehyde (4.39 g, 0.022 mol) was added and stirred at room temperature overnight. The mixture was extracted with hexane/ethyl acetate (1:1), dried over magnesium sulfate and concentrated *in vacuo* to give a white precipitate which was suspended in ether/hexane (1:1) and filtered. The filtrate was concentrated *in vacuo* to afford 7.67 g of the title compound as a pale yellow oil.
¹H NMR (CDCl₃) δ 7.43 (s, 1H), 7.28-7.38 (m, 3H), 7.18-7.25 (m, 2H), 7.12 (tt, J=7.0, 1.1 Hz, 1H), 6.97-7.05 (m, 3H).

### Step 2, 4-(3-phenoxyphenyl)-3-butyn-2-ol

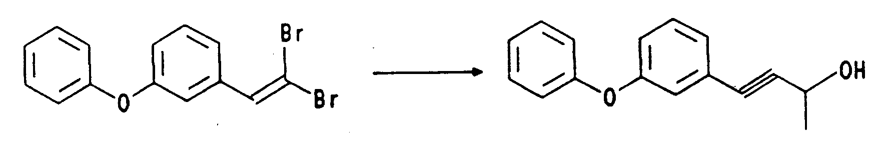

To a solution of the dibromide prepared in Step 1, above, (3.557 g, 10 mmol) in tetrahydrofuran (THF, 20 ml) was added n-butyllithium (n-BuLi, 13.4 ml, 21 mmol) at -78 to -45°C under nitrogen atmosphere. The reaction mixture was stirred at -78°C for 45 minutes and then at room temperature for 1 hour under nitrogen atmosphere. To the mixture was added dry acetaldehyde (about 2 ml) at 0°C and the mixture was stirred at room temperature for 5 minutes. The mixture was combined with 2N HCl (11 ml, 22 mmol), extracted with ethyl acetate and washed with brine, NaHCO₃ solution and then brine again. The solution was dried over magnesium sulfate and concentrated (3.84 g). Chromatography on silica gel eluted with hexane/ethyl acetate (4:1) afforded 2.34 g (75.6% yield) of the title compound as a colorless oil.
¹H NMR (CDCl₃) δ 7.31-7.39 (m, 2H), 7.27 (t, J=7.9 Hz, 1H), 7.09-7.18 (m, 2H), 7.69-7.05 (m, 4H), 4.73 (qd, J=6.6, 5.5 Hz, 1H), 1.86 (d, J=5.5 Hz, 1H), 1.53 (d, J=6.6 Hz, 3H).

### Step 3, N-acetoxy-N-[4-(3-phenoxyphenyl)-3-butyn-2-yl]acetamide

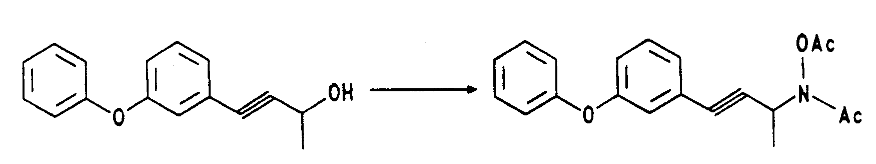

To a solution of the alcohol prepared in Step 2, above, (2 g, 8.4 mmol), N,O-diacetylhydroxylamine, Ph₃P (3.3 g, 12.6 mmol) in toluene (50 ml) and diethyl azodicarboxylate (DEAD, 2.19 g, 12.6 mmol) were added at -78°C under nitrogen atmosphere. The reaction mixture was stirred at room temperature overnight, the resultant precipitate was filtered off and the filtrate was concentrated *in vacuo.* Chromatography on silica gel eluted with hexane/ethyl acetate (4:1) afforded 1.23 g (43% yield) of the title compound as a colorless oil.
¹H NMR (CDCl₃) δ 7.32-7.39 (m, 2H), 7.25 (t, J=7.7 Hz, 1H), 7.09-7.16 (m, 2H), 6.95-7.03 (m, 4H), 5.58 (q, J=6.6 Hz, 1H), 2.23 (s, 3H), 2.05 (s, 3H), 1.48 (d, J=6.6 Hz, 3H).

### Step 4, N-hydroxy-N-[4-[3-phenoxyphenyl)-3-butyn-2-yl]acetamide

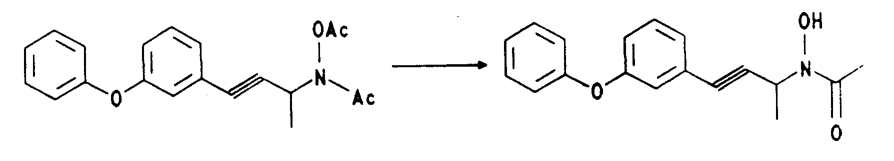

To a solution of the diacetate prepared in Step 3, above, (1.15 g, 3.41 mmol) in methanol (10 ml) was added NH₄OH (2 ml) at room temperature. The mixture was concentrated *in vacuo* and extracted with ethyl acetate. The extract was washed with water and dried over magnesium sulfate. The solution was concentrated to give a colorless oil (1.09 g). Chromatography on silica gel eluted with hexane/ethyl acetate (2:1) afforded the title compound as a white powder. Recrystallization from ethyl acetate/hexane gave 787 mg (78% yield) of product as a white powder.

IR (film) v 3170, 2230, 1600 cm⁻¹.
¹H NMR (DMSO-d₆) δ 7.35-7.46 (m, 3H), 7.15-7.22 (m, 2H), 7.02-7.07 (m, 3H), 6.94-6.97 (m, 1H), 5.47 (q, J=6.9 Hz, 1H), 2.01 (s, 3H), 1.37 (d, J=6.9 Hz, 3H).

### Example 2 N-Hydroxy-N-[4-biphenylyl)-3-butyn-2-yl]acetamide

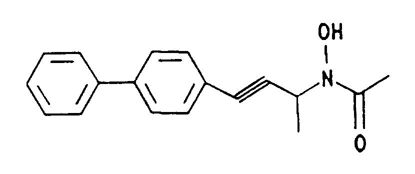

The title compound (m.p. 152.9-153.8°C) was prepared from 4-phenylbenzaldehyde using the same procedure as Example 1.

IR (KBr) v 3450, 1612 cm⁻¹.
¹H NMR (DMSO-d₆) δ 9.90 (s, 1H), 7.66-7.72 (m, 4H), 7.33-7.54 (m, 5H), 5.53 (q, J=7 Hz, 1H), 2.04 (s, 3H), 1.42 (d, J=7 Hz, 3H).

### Example 3 N-Hydroxy-N-[4-(3-phenoxyphenyl)-3-butyn-2-yl]urea

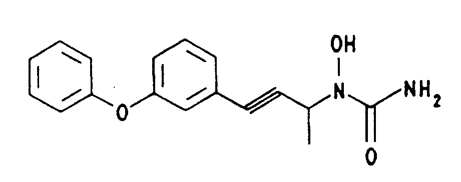

### Step 1, N,O-bis-Boc-(4-phenoxyphenyl-3-butyn-2-yl)hydroxylamine

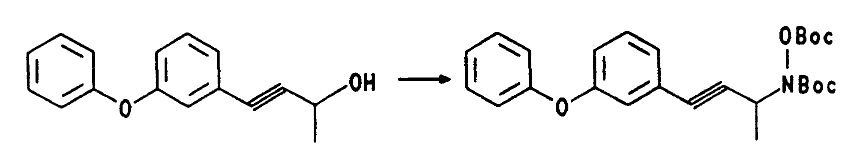

To a solution of 4-phenoxyphenyl-3-butyn-2-ol (1.2 g, 5.04 mmol), Ph₃P (1.98 g, 7.56 mmol) and BocNH-OBoc (1.23 g, 5.29 mmol) in toluene (10 ml) was added DEAD (1.32 g, 7.56 mmol) in toluene (3 ml) at -78°C under nitrogen atmosphere. The mixture was concentrated *in vacuo* and a mixture of hexane/toluene (1:1, 30 ml) was added. The resulting precipitate was separated off by suction filtration and the filtrate was concentrated to give a yellow oil. Chromatography on silica gel eluted with hexane/ethyl acetate (8:1) afforded 1.77 g (77.5% yield) of the desired product as a pale yellow oil. A less pure fraction of the desired product was also obtained (579 mg, 25% yield).

### Step 2, N-hydroxy-N-[4-[3-phenoxynhenyl)-3-butyn-2-yl]urea

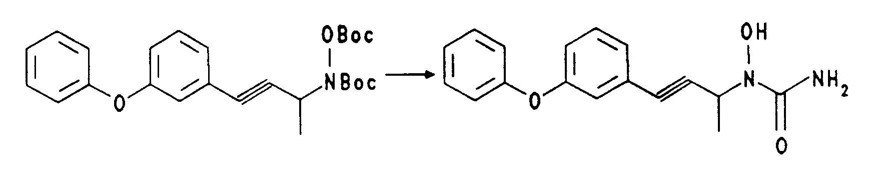

To a solution of the hydroxylamine prepared in Step 1 in CH₂Cl₂ (10 ml) was added trifluoroacetic acid (TFA, 3 ml). The resulting mixture was stirred at room temperature for 1 hour under nitrogen atmosphere, then was washed with small amount of saturated NaHCO₃ solution in brine. The solution was dried over magnesium sulfate and concentrated *in vacuo* to give a pale yellow oil. Without purification, the product was dissolved in THF (10 ml), added to TMS-NCO (731 µl, 5.4 mmol), stirred for 1 hour and concentrated *in vacuo.* Chromatography on silica gel eluted with CH₂Cl₂/acetone (10:1) afforded 610 mg of product as a white powder. This was recrystallized from ethyl acetate/hexane-IPE (1:1:1) to give 456 mg (42.7% yield) of the desired product as a white powder, m.p. 120.0-120.8°C.

IR (KBr) v 3150, 1670, 1580 cm⁻¹.
¹H NMR (CDCl₃) δ 7.31-7.39 (m, 2H), 7.25 (t, J=7.7 Hz, 1H), 7.09-7.18 (m, 2H), 7.04-7.06 (m, 1H), 6.95-7.02 (m, 3H), 6.22 (s, 1H), 5.39 (br s, 2H), 5.34 (q, J=6.9 Hz, 1H), 1.50 (d, J=6.9 Hz, 3H).

### Example 4 N-Hydroxy-N-[4-(4-biphenylyl)-3-butyn-2-yl]urea

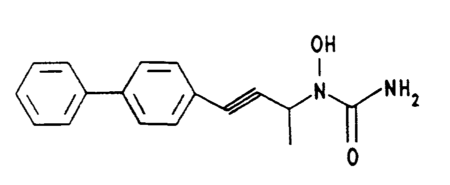

The title compound (m.p. 170.3-171.2°C) was prepared from 4-phenylbenzaldehyde using the same procedure as Example 3.

IR (KBr) v 3460, 1610, 1575 cm⁻¹.
¹H NMR (DMSO-d₆) δ 9.36 (s, 1H), 7.65-7.72 (m, 4H), 7.44-7.52 (m, 4H), 7.38 (tt, J=7.3, 1.5 Hz, 1H), 6.55 (s, 1H), 5.16 (q, J=7.3 Hz, 1H), 1.38 (d, J=6.9 Hz, 3H).

### Example 5 N-Hydroxy-N-[4-(2-naphthyl)-3-butyn-2-yl]urea

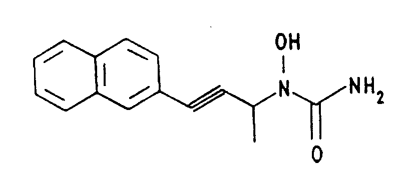

The title compound (m.p. 137.6-138.6°C) was prepared from 2-naphthaldehyde using the same procedure as Example 3.

IR (KBR) v 3450, 3250, 1630 cm⁻¹.
¹H NMR (CDCl₃-DMSO-d₆) δ 9.24 (s, 1H), 7.96 (s, 1H), 7.67-7.87 (m, 3H), 7.38-7.55 (m, 3H), 5.78 (s, 2H), 5.38 (q, J=6.8 Hz, 1H), 1.54 (d, J=6.9 Hz, 3H).

### Example 6 N-Hydroxy-N-[4-(3-allyloxyphenyl)-3-butyn-2-yl]urea

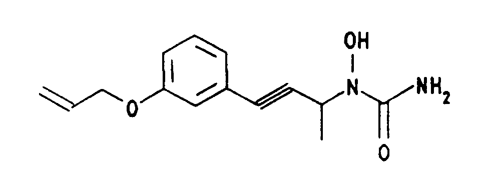

The title compound (m.p. 116.3-116.7°C) was prepared from 3-allyloxybenzaldehyde using the same procedure as Example 3.

IR (KBr) v 3450, 3330, 1630, 1595 cm⁻¹.
¹H NMR (DMSO-d₆) δ 7.26 (t, J=8.4 Hz, 1H), 6.90-6.99 (m, 2H), 6.52 (br s, 2H), 5.95-6.10 (m, 1H), 5.38 (dq, J=16, 1 Hz, 1H), 5.26 (dq, J=9.5, 1 Hz, 1H), 4.54-4.60 (m, 2H), 1.35 (d, J=7 Hz, 3H).

### Example 7 N-Hydroxy-N-[4-(3-cyclohexyloxyphenyl)-3-butyn-2-yl]urea

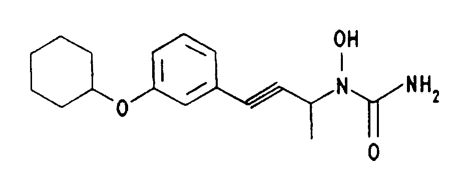

### Step 1, 3-cyclohexyloxy-1-iodobenzene

A solution of cyclohexanol (6.0 g), 3-iodophenol (13.2 g) and triphenylphosphine (15.7 g) in THF (150 ml) was stirred at room temperature under nitrogen atmosphere. Diethyl azodicarboxylate (10.45 g) was added dropwise and the resulting solution was stirred for 7 days. The reaction mixture was evaporated to dryness under reduced pressure, hexane/ether (2:1) mixture (400 ml) was added to the precipitate, the undissolved precipitate was filtered off and the filtrate was concentrated *in vacuo.* The crude product was purified by chromatography on silica gel (ether/hexane, 1:4), and distillation (200°C, 0.4 mmHg, bath temp.) to provide 7.46 g (41% yield) of title product.

### Step 2, N,O-bis-tert-butoxycarbonyl-N-[4-(3-cyclohexyloxyphenyl)-3-butyn-2-yl]hydroxylamine and N-tert-butoxycarbonyl]-N-[4-(3-cyclohexyloxyphenyl)-3-butyn-2-yl]hydroxylamine

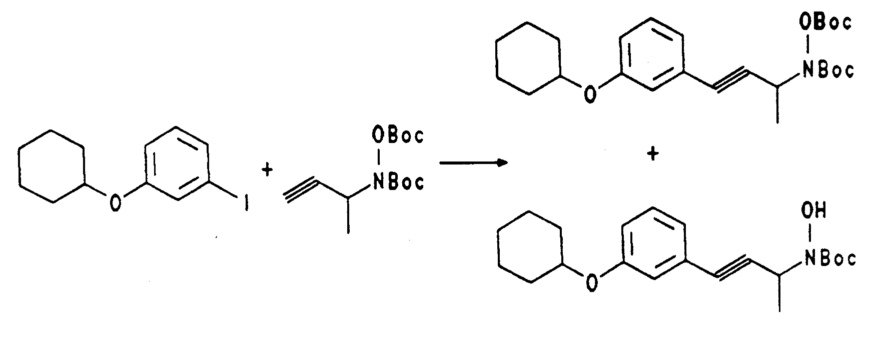

Diethylamine (25 ml), suspension of Cul (0.043 g) and (Ph₃P)₂PdCl₂ (0.33 g) was stirred at room temperature under argon atmosphere. Diethylamine solution of N,O-bis-*tert*-butoxycarbonyl-N-(3-butyn-2-yl)hydroxylamine (5.42 g) was added to the suspension, then the 3-cyclohexyloxy-1-iodobenzene (4.53 g) prepared in Step 1 was added. The resulting mixture was stirred for 17 hours. Solvent was removed under reduced pressure and ether (200 ml) and water (100 ml) were added. Undissoluble red grease was filtered off (celite). Ethereal layer of the filtrate was collected, dried (MgSO₄), and concentrated under reduced pressure. The crude product was purified by silica gel column chromatography (ether/hexane, 15:85), providing 4.0 g (59 % yield) of N,O-bis-*tert*-butoxycarbonyl-N-[4-(3-cyclohexyloxyphenyl)-3-butyn-2-yl]hydroxylamine and 1.6 g (30% yield) of N-*tert-*butoxycarbonyl]-N-[4-(3-cyclohexyloxyphenyl)-3-butyn-2-yl]hydroxylamine.

### Step 3, N-hydroxy-N-[4-(3-cyclohexyloxyphenyl)-3-butyn-2-yl]urea

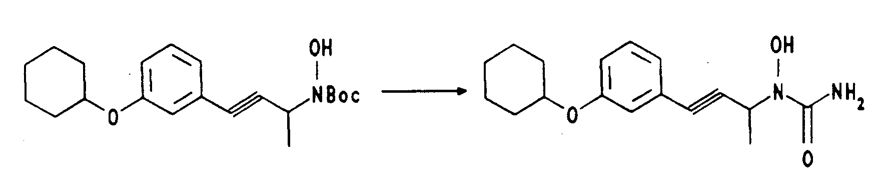

The N-*tert*-butoxycarbonyl (1.51 g) prepared in Step 2 was dissolved in CH₂Cl₂ (30 ml). CF₃CO₂H (4 ml) was added to the solution at room temperature and the reaction mixture was stirred for 4 hours and concentrated under reduced pressure. NaHCO₃ solution was added and extracted with CH₂Cl₂. The organic layer was dried (MgSO₄) and concentrated *in vacuo.* The resulting crude product was dissolved in dry THF (10 ml). Trimethylsilylisocyanate (1.09 g) was added to the solution, the mixture was stirred for 3 hours and the solvent was removed *in vacuo.* The resulting crude product was recrystallized with CHCl₃/hexane, affording 0.62 g (45% yield) of the title compound (m.p. 95-95.5°C).

IR (KBr) v 3405, 1668 cm⁻¹.
¹H NMR (CDCl₃) δ 7.17 (dd, J=7.7, 7.7 Hz, 1H), 6.9-7.0 (m, 2H), 6.86 (ddd, J=8.4, 2.6, 1.1 Hz, 1H), 5.35 (q, J=7.0 Hz, 1H), 4.17-4.25 (m, 1H), 1.92-1.98 (m, 2H), 1.77-1.80 (m, 2H), 1.50 (d, J=7.0 Hz, 3H), 1.31-1.58 (m, 6H).

### Example 8 N-Hydroxy-N-[4-(3-cyclohexyloxyphenyl)-3-butyn-2-yl]acetamide

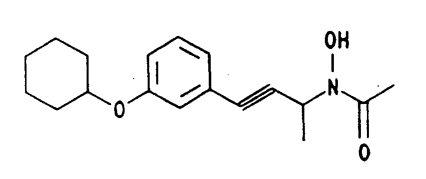

### Step 1, N,O-diacetyl-N-[4-(3-cyclohexyloxyphenyl)-3-butyn-2-yl]hydroxylamine

N,O-Di-*tert*-butoxycarbonyl-N-[4-(3-cyclohexyloxyphenyl)-3-butyn-2-yl]hydroxylamine (2.0 g) was dissolved in CH₂Cl₂ (15 ml). CF₃CO₂H (4 ml) was added to the solution at room temperature and the reaction mixture was stirred for 2 hours and concentrated under reduced pressure. The crude product was dissolved in CH₂Cl₂ (20 ml) and stirred, then pyridine (5 ml) and AcCl (2.5 ml) were added at 0°C and stirred for 2 hours. The solvent was removed under reduced pressure, the residue was combined with 10% citric acid and extracted with ether. The organic layer was washed with NaHCO₃ solution, dried (MgSO₄) and concentrated *in vacuo.* The crude product was purified by silica gel column chromatography (hexane/EtOAc, 2:1), affording the title product as a colorless oil.

### Step 2, N-hydroxy-N-[4-(3-cyclohexyloxyphenyl)-3-butyn-2-yl]acetamide

To a stirred solution of the product of Step 1, above (1.1 g), in MeOH (15 ml) was added 25% NH₃ solution (3 ml) at room temperature. The reaction mixture was stirred for 30 minutes and the solvent was evaporated off. To the resulting mixture water was added and the mixture was extracted with ether. The extract was dried over magnesium sulfate and concentrated *in vacuo.* The crude product was purified by silica gel column chromatography (hexane/EtOAc, 2:1), affording the title compound (0.412 g) as a colorless oil.

IR (neat) v 2940, 1617, 1605, 1422, 1282 cm⁻¹.
¹H NMR (DMSO) δ 9.9 (br, 1H), 7.25 (dd, J=7.3, 8.8 Hz, 1H), 6.93-6.98 (m, 3H), 5.48 (q, J=7.0 Hz, 1H), 4.35 (m, 1H), 2.03 (s, 3H), 1.24-1.87 (m, 10H), 1.36 (d, J=7.0 Hz, 3H).

## Claims (Claims for the following Contracting State(s): AT, BE, CH, LI, DE, DK, FR, GB, IT, LU, NL, SE,)

1. A process for preparing a compound of the formula 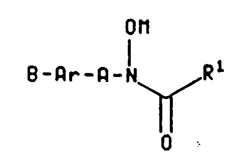 wherein R¹ is hydrogen, C1 to C4 alkyl, C2 to C4 alkenyl, alkylthioalkyl, alkoxyalkyl or NR²R³;
R² and R³ are each independently hydrogen, C1 to C4 alkyl, hydroxy, aryl, or aryl substituted with one or more substituents selected from the group consisting of halo, nitro, cyano, C1 to C12 alkyl, C1 to C12 alkoxy, C1 to C12 halosubstituted alkyl, C1 to C12 hydroxysubstituted alkyl, C1 to C12 alkoxycarbonyl, aminocarbonyl, C1 to C12 alkylaminocarbonyl, di C1 to C12 alkylaminocarbonyl and C1 to C12 alkylsulfonyl, wherein R² and R³ are not both hydroxy;
M is hydrogen, a pharmaceutically acceptable cation, aroyl or C1 to C12 alkoyl;
A is alkynylene or alkynylene substituted with one or more substituents selected from the group consisting of C1 to C4 alkyl, C2 to C4 alkenyl, cyano, halo, C1 to C4 alkoxy, C1 to C4 halosubstituted alkyl and C1 to C4 hydroxysubstituted alkyl;
Ar is phenyl or naphthyl;
B is hydrogen, C1 to C12 alkyl, C2 to C12 alkenyl, C1 to C12 halosubstituted alkyl, C1 to C12 hydroxysubstituted alkyl, OR⁴, NR⁴R⁵, aryl or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl;
R⁴ and R⁵ are each independently hydrogen, C1 to C12 alkyl, C3 to C12 alkenyl, C3 to C8 cycloalkyl, aryl or 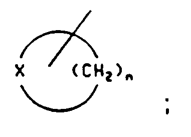 n is 4 to 6; X is O, NR⁶ or S; and
R⁶ is hydrogen or C1 to C4 alkyl; comprising:
(I) selectively hydrolyzing a compound having the formula 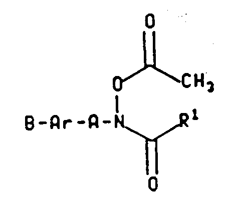 wherein A, B, Ar and R¹ are as defined above, with a base selected from ammonium hydroxide, sodium hydroxide, potassium hydroxide and lithium hydroxide in a solvent system under conditions including a reaction temperature of between -10°C and ambient temperature;
(II) reaction of a compound having the formula 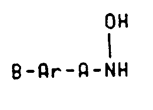 wherein A, B, Ar and R¹ are as defined above, with trimethylsilyl isocyanate in a reaction-inert solvent under conditions including a reaction temperature of between ambient and reflux temperature; or
(III) reaction of a compound having the formula 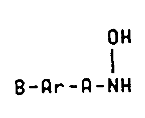 wherein A, B, Ar and R¹ are as defined above, with gaseous hydrogen chloride in a reaction-inert solvent under reaction conditions including a reaction temperature of between ambient temperature and boiling point of the solvent, followed by treatment with phosgene.

2. A process according to Claim 1 wherein:
when process (I) is used, said solvent system is selected from one or more of water, methanol, ethanol, propanol and tetrahydrofuran;
when process (II) is used, said reaction-inert solvent is selected from tetrahydrofuran, dioxane, methylene chloride and benzene; and
when process (III) is used, said reaction-inert solvent is selected from benzene and toluene.

3. A process according to Claim 1 or 2 further comprising the step of isolating said prepared compound.

4. A process according to one of Claims 1 to 3 wherein:
when process (I) is used, R¹ is C1 to C4 alkyl; and
when process (II) or (III) is used, R¹ is NR²R³.

5. A process according to Claim 4 wherein:
when process (I) is used, R¹ is methyl; and
when process (II) or (III) is used, R¹ is NH₂.

6. A process according to one of Claims 1 to 5, wherein process (III) is used, further comprising the step of treating the reaction mixture with aqueous ammonia.

7. A compound of the formula 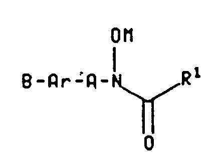 wherein R¹ is hydrogen, C1 to C4 alkyl, C2 to C4 alkenyl, alkylthioalkyl, alkoxyalkyl or NR²R³;
R² and R³ are each independently hydrogen, C1 to C4 alkyl, hydroxy, aryl, or aryl substituted with one or more substituents selected from the group consisting of halo, nitro, cyano, C1 to C12 alkyl, C1 to C12 alkoxy, C1 to C12 halosubstituted alkyl, C1 to C12 hydroxysubstituted alkyl, C1 to C12 alkoxycarbonyl, aminocarbonyl, C1 to C12 alkylaminocarbonyl, di C1 to C12 alkylaminocarbonyl and C1 to C12 alkylsulfonyl, with the proviso that R² and R³ are not both hydroxy;
M is hydrogen, a pharmaceutically acceptable cation, aroyl or C1 to C12 alkoyl;
A is alkynylene or alkynylene substituted with one or more substituents selected from the group consisting of C1 to C4 alkyl, C2 to C4 alkenyl, cyano, halo, C1 to C4 alkoxy, C1 to C4 halosubstituted alkyl and C1 to C4 hydroxysubstituted alkyl;
Ar is phenyl or naphthyl;
B is hydrogen, C1 to C12 alkyl, C2 to C12 alkenyl, C1 to C12 halosubstituted alkyl, C1 to C12 hydroxysubstituted alkyl, OR⁴, NR⁴R⁵, aryl or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl;
R⁴ and R⁵ are each independently hydrogen, C1 to C12 alkyl, C3 to C12 alkenyl, C3 to C8 cycloalkyl, aryl or 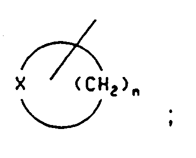 n is 4 to 6;
X is O, NR⁶ or S; and
R⁶ is hydrogen or C1 to C4 alkyl.

8. A compound according to Claim 7 wherein:
A is alkynylene;
R¹ is C1 to C4 alkyl; and
B is hydrogen, aryl, or aryloxy.

9. A compound according to Claim 7 wherein:
A is alkynylene;
R¹ is NR²R³; and
B is hydrogen, aryl, aryloxy or C3 to C12 alkenyloxy.

10. A compound according to one of Claims 7 to 9 wherein Ar is phenyl.

11. A compound according to Claim 7 or 8 wherein:
Ar is phenyl;
M is hydrogen; and
R¹ is methyl.

12. A compound according to Claim 7 or 9 wherein:
Ar is phenyl;
M is hydrogen; and
R¹ is amino.

13. A compound according to one of Claims 7 to 12 wherein B is phenyl or phenoxy.

14. A compound according to one of Claims 7, 9 and 12 wherein B is allyloxy.

15. A compound according to one of Claims 7 to 9 wherein:
Ar is naphthyl; and
B is hydrogen.

16. A pharmaceutical composition comprising a compound according to one of Claims 7 to 15, or a pharmaceutically acceptable salt thereof, and a pharmaceutically acceptable carrier.

17. A compound according to any one of claims 7 to 15, or a pharmaceutically acceptable salt thereof, for use as a medicament.

18. The use of a compound according to any one of claims 7 to 15, or of a pharmaceutically acceptable salt thereof, for the manufacture of a medicament for treating allergic or inflammatory conditions, or cardiovascular diseases, in a mammal, including a human being.

## Claims (Claims for the following Contracting State(s): ES, GR)

1. A process for preparing a compound of the formula 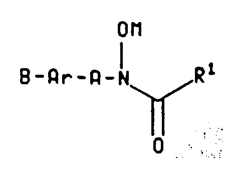 wherein R¹ is hydrogen, C1 to C4 alkyl, C2 to C4 alkenyl, alkylthioalkyl, alkoxyalkyl or NR²R³;
R² and R³ are each independently hydrogen, C1 to C4 alkyl, hydroxy, aryl, or aryl substituted with one or more substituents selected from the group consisting of halo, nitro, cyano, C1 to C12 alkyl, C1 to C12 alkoxy, C1 to C12 halosubstituted alkyl, C1 to C12 hydroxysubstituted alkyl, C1 to C12 alkoxycarbonyl, aminocarbonyl, C1 to C12 alkylaminocarbonyl, di C1 to C12 alkylaminocarbonyl and C1 to C12 alkylsulfonyl, wherein R² and R³ are not both hydroxy;
M is hydrogen, a pharmaceutically acceptable cation, aroyl or C1 to C12 alkoyl;
A is alkynylene or alkynylene substituted with one or more substituents selected from the group consisting of C1 to C4 alkyl, C2 to C4 alkenyl, cyano, halo, C1 to C4 alkoxy, C1 to C4 halosubstituted alkyl and C1 to C4 hydroxysubstituted alkyl;
Ar is phenyl or naphthyl;
B is hydrogen, C1 to C12 alkyl, C2 to C12 alkenyl, C1 to C12 halosubstituted alkyl, C1 to C12 hydroxysubstituted alkyl, OR⁴, NR⁴R⁵, aryl or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl;
R⁴ and R⁵ are each independently hydrogen, C1 to C12 alkyl, C3 to C12 alkenyl, C3 to C8 cycloalkyl, aryl or 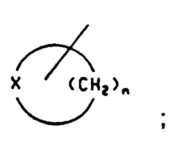 n is 4 to 6; X is O, NR⁶ or S; and
R⁶ is hydrogen or C1 to C4 alkyl; comprising:
(I) selectively hydrolyzing a compound having the formula 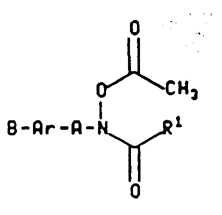 wherein A, B, Ar and R¹ are as defined above, with a base selected from ammonium hydroxide, sodium hydroxide, potassium hydroxide and lithium hydroxide in a solvent system under conditions including a reaction temperature of between -10°C and ambient temperature;
(II) reaction of a compound having the formula 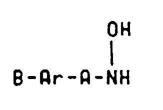 wherein A, B, Ar and R¹ are as defined above, with trimethylsilyl isocyanate ir a reaction-inert solvent under conditions including a reaction temperature of between ambient and reflux temperature; or
(III) reaction of a compound having the formula 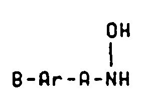 wherein A, B, Ar and R¹ are as defined above, with gaseous hydrogen chloride in a reaction-inert solvent under reaction conditions including a reaction temperature of between ambient temperature and boiling point of the solvent, followed by treatment with phosgene.

2. A process according to Claim 1 wherein:
when process (I) is used, said solvent system is selected from one or more of water, methanol, ethanol, propanol and tetrahydrofuran;
when process (II) is used; said reaction-inert solvent is selected from tetrahydrofuran, dioxane, methylene chloride and benzene; and
when process (III) is used, said reaction-inert solvent is selected from benzene and toluene.

3. A process according to Claim 1 or 2 further comprising the step of isolating said prepared compound.

4. A process according to one of Claims 1 to 3 wherein:
when process (I) is used, R¹ is C1 to C4 alkyl; and
when process (II) or (III) is used, R¹ is NR²R³.

5. A process according to Claim 4 wherein:
when process (I) is used, R¹ is methyl; and
when process (II) or (III) is used, R¹ is NH₂.

6. A process according to one of Claims 1 to 5, wherein process (III) is used, further comprising the step of treating the reaction mixture with aqueous ammonia.

## Patentansprüche (Patentansprüche für folgende(n) Vertragsstaat(en): AT, BE, CH, LI, DE, DK, FR, GB, IT, LU, NL, SE)

1. Verfahren zur Herstellung einer Verbindung der Formel 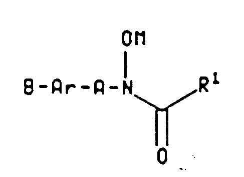 worin bedeuten:
R¹ Wasserstoff, C1- bis C4-Alkyl, C2- bis C4-Alkenyl, Alkylthioalkyl, Alkoxyalkyl oder NR²R³,
R² und R³ jeweils unabhängig voneinander Wasserstoff, C1- bis C4-Alkyl, Hydroxy, Aryl oder Aryl, substituiert mit einem oder mehreren Substituenten, ausgewählt aus der Gruppe Halogen, Nitro, Cyano, C1- bis C12-Alkyl, C1- bis C12-Alkoxy, C1- bis C12-halogensubstituiertem Alkyl, C1- bis C12-hydroxysubstituiertem Alkyl, C1- bis C12-Alkoxycarbonyl, Aminocarbonyl, C1- bis C12-Alkylaminocarbonyl, Di-C1- bis C12-alkylaminocarbonyl und C1- bis C12-Alkylsulfonyl, wobei R² und R³ nicht beide gleichzeitig für Hydroxy stehen dürfen,
M Wasserstoff, ein pharmazeutisch akzeptables Kation, Aroyl oder C1- bis C12-Alkoyl,
A Alkinylen oder Alkinylen, substituiert mit einem oder mehreren Substituenten, ausgewählt aus der Gruppe C1- bis C4-Alkyl, C2- bis C4-Alkenyl, Cyano, Halogen, C1- bis C4-Alkoxy, C1- bis C4-halogensubstituiertem Alkyl und C1- bis C4-hydroxysubstituiertem Alkyl,
Ar Phenyl oder Naphthyl,
B Wasserstoff, C1- bis C12-Alkyl, C2- bis C12-Alkenyl, C1- bis C12-halogensubstituiertes Alkyl, C1- bis C12-hydroxysubstituiertes Alkyl, OR⁴, NR⁴R⁵, Aryl oder Aryl, substituiert mit einem oder mehreren Substituenten, ausgewählt aus der Gruppe Halogen, Nitro, Cyano, C1- bis C6-Alkyl, C1- bis C6-Alkoxy, C1- bis C6-halogensubstituiertem Alkyl, C1- bis C6-hydroxysubstituiertem Alkyl, C1- bis C6-Alkoxycarbonyl, Aminocarbonyl, C1- bis C6-Alkylaminocarbonyl, Di-C1- bis C6-alkylaminocarbonyl und C1- bis C6-Alkylsulfonyl,
R⁴ und R⁵ jeweils unabhängig voneinander Wasserstoff, C1- bis C12-Alkyl, C3- bis C12-Alkenyl, C3- bis C8-Cycloalkyl, Aryl oder 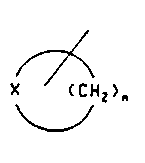 n = 4 bis 6 und X = O, NR⁶ oder S und
R⁶ Wasserstoff oder C1- bis C4-Alkyl,
durch
(I) selektives Hydrolysieren einer Verbindung der Formel 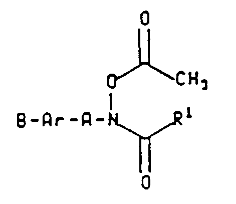 worin A, B, Ar und R¹ die oben angegebene Bedeutung besitzen, mit einer Base, ausgewählt aus Ammoniumhydroxid, Natriumhydroxid, Kaliumhydroxid und Lithiumhydroxid, in einem Lösungsmittelsystem unter Bedingungen, einschließlich einer Reaktionstemperatur zwischen -10°C und Raumtemperatur,
(II) eine Reaktion einer Verbindung der Formel 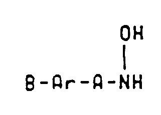 worin A, B, Ar und R¹ die oben angegebene Bedeutung besitzen, mit Trimethylsilylisocyanat in einem reaktionsinerten Lösungsmittel unter Bedingungen, einschließlich einer Reaktionstemperatur zwischen Umgebungstemperatur und Rückflußtemperatur, oder
(III) eine Reaktion einer Verbindung der Formel 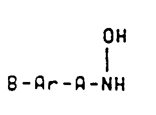 worin A, B, Ar und R¹ die oben angegebene Bedeutung besitzen, mit gasförmigem Chlorwasserstoff in einem reaktionsinerten Lösungsmittel unter Reaktionsbedingungen, einschließlich einer Reaktionstemperatur zwischen Umgebungstemperatur und dem Siedepunkt des Lösungsmittels, und eine anschließende Behandlung mit Phosgen.

2. Verfahren nach Anspruch 1, wobei bei Verwendung des Verfahrens (I) das Lösungsmittelsystem aus einem oder mehreren der Gruppe Wasser, Methanol, Ethanol, Propanol und Tetrahydrofuran ausgewählt ist, bei Verwendung des Verfahrens (II) das reaktionsinerte Lösungsmittel aus Tetrahydrofuran, Dioxan, Methylenchlorid und Benzol ausgewählt ist und bei Verwendung des Verfahrens (III) das reaktionsinerte Lösungsmittel aus Benzol und Toluol ausgewählt ist.

3. Verfahren nach Anspruch 1 oder 2, des weiteren umfassend den Schritt eines Isolierens der hergestellten Verbindung.

4. Verfahren nach einem der Ansprüche 1 bis 3, wobei bei Verwendung des Verfahrens (I) R¹ für C1- bis C4-Alkyl steht und
bei Verwendung des Verfahrens (II) oder (III) R¹ für NR²R³ steht.

5. Verfahren nach Anspruch 4, wobei
bei Verwendung des Verfahrens (I) R¹ für Methyl steht und
bei Verwendung des Verfahrens (II) oder (III) R¹ für NH₂ steht.

6. Verfahren nach einem der Ansprüche 1 bis 5, wobei das Verfahren (III) verwendet wird, des weiteren umfassend den Schritt eines Behandelns des Reaktionsgemisches mit wäßrigem Ammoniak.

7. Verbindung der Formel 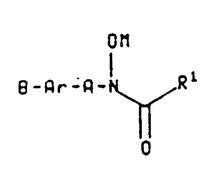 worin bedeuten:
R¹ Wasserstoff, C1- bis C4-Alkyl, C2- bis C4-Alkenyl, Alkylthioalkyl, Alkoxyalkyl oder NR²R³,
R² und R³ jeweils unabhängig voneinander Wasserstoff, C1- bis C4-Alkyl, Hydroxy, Aryl oder Aryl, substituiert mit einem oder mehreren Substituenten, ausgewählt aus der Gruppe Halogen, Nitro, Cyano, C1- bis C12-Alkyl, C1- bis C12-Alkoxy, C1- bis C12-halogensubstituiertem Alkyl, C1- bis C12-hydroxysubstituiertem Alkyl, C1- bis C12-Alkoxycarbonyl, Aminocarbonyl, C1- bis C12-Alkylaminocarbonyl, Di-C1- bis C12-alkylaminocarbonyl und C1- bis C12-Alkylsulfonyl, wobei gilt, daß R² und R³ nicht beide gleichzeitig für Hydroxy stehen dürfen,
M Wasserstoff, ein pharmazeutisch akzeptables Kation, Aroyl oder C1- bis C12-Alkoyl,
A Alkinylen oder Alkinylen, substituiert mit einem oder mehreren Substituenten, ausgewählt aus der Gruppe C1- bis C4-Alkyl, C2- bis C4-Alkenyl, Cyano, Halogen, C1- bis C4-Alkoxy, C1- bis C4-halogensubstituiertem Alkyl und C1- bis C4-hydroxysubstituiertem Alkyl,
Ar Phenyl oder Naphthyl,
B Wasserstoff, C1- bis C12-Alkyl, C2- bis C12-Alkenyl, C1- bis C12-halogensubstituiertes Alkyl, C1- bis C12-hydroxysubstituiertes Alkyl, OR⁴, NR⁴R⁵, Aryl oder Aryl, substituiert mit einem oder mehreren Substituenten, ausgewählt aus der Gruppe Halogen, Nitro, Cyano, C1- bis C6-Alkyl, C1- bis C6-Alkoxy, C1- bis C6-halogensubstituiertem Alkyl, C1- bis C6-hydroxysubstituiertem Alkyl, C1- bis C6-Alkoxycarbonyl, Aminocarbonyl, C1- bis C6-Alkylaminocarbonyl, Di-C1- bis C6-alkylaminocarbonyl und C1- bis C6-Alkylsulfonyl,
R⁴ und R⁵ jeweils unabhängig voneinander Wasserstoff, C1- bis C12-Alkyl, C3- bis C12-Alkenyl, C3- bis C8-Cycloalkyl, Aryl oder 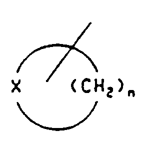 n = 4 bis 6,
X = O, NR⁶ oder S und
R⁶ Wasserstoff oder C1- bis C4-Alkyl.

8. Verbindung nach Anspruch 7, worin bedeuten:
A Alkinylen;
R¹ C1- bis C4-Alkyl und
B Wasserstoff, Aryl oder Aryloxy.

9. Verbindung nach Anspruch 7, worin bedeuten:
A Alkinylen,
R¹ NR²R³ und
B Wasserstoff, Aryl, Aryloxy oder C3- bis C12-Alkenyloxy.

10. Verbindung nach einem der Ansprüche 7 bis 9, worin Ar für Phenyl steht.

11. Verbindung nach Anspruch 7 oder 8, worin bedeuten:
Ar Phenyl,
M Wasserstoff und
R¹ Methyl.

12. Verbindung nach Anspruch 7 oder 9, worin bedeuten:
Ar Phenyl,
M Wasserstoff und
R¹ Amino.

13. Verbindung nach einem der Ansprüche 7 bis 12, worin B für Phenyl oder Phenoxy steht.

14. Verbindung nach einem der Ansprüche 7, 9 und 12, worin B für Allyloxy steht.

15. Verbindung nach einem der Ansprüche 7 bis 9, worin bedeuten:
Ar Naphthyl und
B Wasserstoff.

16. Arzneimittelzubereitung mit einer Verbindung nach einem der Ansprüche 7 bis 15 oder einem pharmazeutisch akzeptablen Salz derselben und einem pharmazeutisch akzeptablen Träger.

17. Verbindung nach einem der Ansprüche 7 bis 15 oder ein pharmazeutisch akzeptables Salz derselben zur Verwendung als Medikament.

18. Verwendung einer Verbindung nach einem der Ansprüche 7 bis 15 oder eines pharmazeutisch akzeptablen Salzes derselben zur Herstellung eines Medikamentes zur Behandlung allergischer Zustände oder von Entzündungszuständen oder kardiovaskulärer Erkrankungen bei einem Säugetier, einschließlich Menschen.

## Patentansprüche (Patentansprüche für folgende(n) Vertragsstaat(en): ES, GR)

1. Verfahren zur Herstellung einer Verbindung der Formel 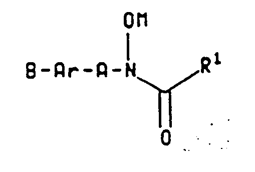 worin bedeuten:
R¹ Wasserstoff, C1- bis C4-Alkyl, C2- bis C4-Alkenyl, Alkylthioalkyl, Alkoxyalkyl oder NR²R³,
R² und R³ jeweils unabhängig voneinander Wasserstoff, C1- bis C4-Alkyl, Hydroxy, Aryl oder Aryl, substituiert mit einem oder mehreren Substituenten, ausgewählt aus der Gruppe Halogen, Nitro, Cyano, C1- bis C12-Alkyl, C1- bis C12-Alkoxy, C1- bis C12-halogensubstituiertem Alkyl, C1- bis C12-hydroxysubstituiertem Alkyl, C1- bis C12-Alkoxycarbonyl, Aminocarbonyl, C1- bis C12-Alkylaminocarbonyl, Di-C1- bis C12-alkylaminocarbonyl und C1- bis C12-Alkylsulfonyl, wobei R² und R³ nicht beide gleichzeitig für Hydroxy stehen dürfen,
M Wasserstoff, ein pharmazeutisch akzeptables Kation, Aroyl oder C1- bis C12-Alkoyl,
A Alkinylen oder Alkinylen, substituiert mit einem oder mehreren Substituenten, ausgewählt aus der Gruppe C1- bis C4-Alkyl, C2- bis C4-Alkenyl, Cyano, Halogen, C1- bis C4-Alkoxy, C1- bis C4-halogensubstituiertem Alkyl und C1- bis C4-hydroxysubstituiertem Alkyl,
Ar Phenyl oder Naphthyl,
B Wasserstoff, C1- bis C12-Alkyl, C2- bis C12-Alkenyl, C1- bis C12-halogensubstituiertes Alkyl, C1- bis C12-hydroxysubstituiertes Alkyl, OR⁴, NR⁴R⁵, Aryl oder Aryl, substituiert mit einem oder mehreren Substituenten, ausgewählt aus der Gruppe Halogen, Nitro, Cyano, C1- bis C6-Alkyl, C1- bis C6-Alkoxy, C1- bis C6-halogensubstituiertem Alkyl, C1- bis C6-hydroxysubstituiertem Alkyl, C1- bis C6-Alkoxycarbonyl, Aminocarbonyl, C1- bis C6-Alkylaminocarbonyl, Di-C1- bis C6-alkylaminocarbonyl und C1- bis C6-Alkylsulfonyl,
R⁴ und R⁵ jeweils unabhängig voneinander Wasserstoff, C1- bis C12-Alkyl, C3- bis C12-Alkenyl, C3- bis C8-Cycloalkyl, Aryl oder 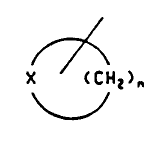 n = 4 bis 6 und X = O, NR⁶ oder S und
R⁶ Wasserstoff oder C1- bis C4-Alkyl,
durch
(I) selektives Hydrolysieren einer Verbindung der Formel 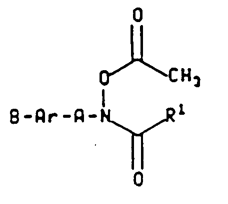 worin A, B, Ar und R¹ die oben angegebene Bedeutung besitzen, mit einer Base, ausgewählt aus Ammoniumhydroxid, Natriumhydroxid, Kaliumhydroxid und Lithiumhydroxid, in einem Lösungsmittelsystem unter Bedingungen, einschließlich einer Reaktionstemperatur zwischen -10°C und Raumtemperatur,
(II) eine Reaktion einer Verbindung der Formel 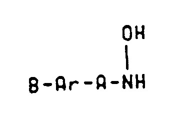 worin A, B, Ar und R¹ die oben angegebene Bedeutung besitzen, mit Trimethylsilylisocyanat in einem reaktionsinerten Lösungsmittel unter Bedingungen, einschließlich einer Reaktionstemperatur zwischen Umgebungstemperatur und Rückflußtemperatur, oder
(III) eine Reaktion einer Verbindung der Formel 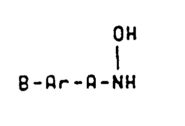 worin A, B, Ar und R¹ die oben angegebene Bedeutung besitzen, mit gasförmigem Chlorwasserstoff in einem reaktionsinerten Lösungsmittel unter Reaktionsbedingungen, einschließlich einer Reaktionstemperatur zwischen Umgebungstemperatur und dem Siedepunkt des Lösungsmittels, und eine anschließende Behandlung mit Phosgen.

2. Verfahren nach Anspruch 1, wobei bei Verwendung des Verfahrens (I) das Lösungsmittelsystem aus einem oder mehreren der Gruppe Wasser, Methanol, Ethanol, Propanol und Tetrahydrofuran ausgewählt ist, bei Verwendung des Verfahrens (II) das reaktionsinerte Lösungsmittel aus Tetrahydrofuran, Dioxan, Methylenchlorid und Benzol ausgewählt ist und bei Verwendung des Verfahrens (III) das reaktionsinerte Lösungsmittel aus Benzol und Toluol ausgewählt ist.

3. Verfahren nach Anspruch 1 oder 2, des weiteren umfassend den Schritt eines Isolierens der hergestellten Verbindung.

4. Verfahren nach einem der Ansprüche 1 bis 3, wobei bei Verwendung des Verfahrens (I) R¹ für C1- bis C4-Alkyl steht und
bei Verwendung des Verfahrens (II) oder (III) R¹ für NR²R³ steht.

5. Verfahren nach Anspruch 4, wobei
bei Verwendung des Verfahrens (I) R¹ für Methyl steht und
bei Verwendung des Verfahrens (II) oder (III) R¹ für NH₂ steht.

6. Verfahren nach einem der Ansprüche 1 bis 5, wobei Verfahren (III) verwendet wird, des weiteren umfassend den Schritt eines Behandelns des Reaktionsgemisches mit wäßrigem Ammoniak.

## Revendications (Revendications pour l'(les) Etat(s) contractant(s) suivant(s): AT, BE, CH, LI, DE, DK, FR, GB, IT, LU, NL, SE)

1. Procédé de préparation d'un composé de formule 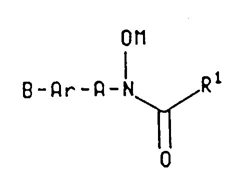 dans laquelle R¹ représente l'hydrogène, un groupe alkyle en C₁-C₄, alkényle en C₂-C₄, alkylthioalkyle, alcoxyalkyle ou NR²R³ ;
R² et R³ représentent chacun indépendamment l'hydrogène, un groupe alkyle en C₁-C₄, hydroxy, aryle ou aryle substitué par un ou plusieurs substituants choisis dans le groupe comprenant les radicaux halogéno, nitro, cyano, alkyle en C₁-C₁₂, alcoxy en C₁-C₁₂, (alkyle en C₁-C₁₂)halogéno-substitué, (alkyle en C₁-C₁₂)hydroxy-substitué, (alcoxy en C₁-C₁₂)carbonyle, aminocarbonyle, (alkyle en C₁-C₁₂)aminocarbonyle, di(alkyle en C₁-C₁₂)aminocarbonyle et (alkyle en C₁-C₁₂)sulfonyle, R² et R³ ne représentant pas simultanément hydroxy ;
M représente l'hydrogène, un cation pharmaceutiquement acceptable, un groupe aroyle ou alkoyle en C₁-C₁₂ ;
A est un groupe alkynylène ou alkynylène substitué par un ou plusieurs substituants choisis dans le groupe comprenant les radicaux alkyle en C₁-C₄, alkényle en C₂-C₄, cyano, halogéno, alcoxy en C₁-C₄, (alkyle en C₁-C₄)halogéno-substitué et (alkyle en C₁-C₄)hydroxy-substitué ;
Ar est un groupe phényle ou naphtyle ;
B représente l'hydrogène, un groupe alkyle en C₁-C₁₂, alkényle en C₂-C₁₂, (alkyle en C₁-C₁₂)halogéno-substitué, (alkyle en C₁-C₁₂)hydroxy-substitué, OR⁴, NR⁴R⁵, aryle ou aryle substitué par un ou plusieurs substituants choisis dans le groupe comprenant les radicaux halogéno, nitro, cyano, alkyle en C₁-C₆, alcoxy en C₁-C₆, (alkyle en C₁-C₆)halogéno-substitué, (alkyle en C₁-C₆)hydroxy-substitué, (alcoxy en C₁-C₆)carbonyle, aminocarbonyle,(alkyle en C₁-C₆)aminocarbonyle, di(alkyle en C₁-C₆)aminocarbonyle et (alkyle en C₁-C₆)sulfonyle ;
R⁴ et R⁵ représentent chacun indépendamment l'hydrogène, un groupe alkyle en C₁-C₁₂, un groupe alkényle en C₃-C₁₂, cycloalkyle en C₃-C₈, aryle ou 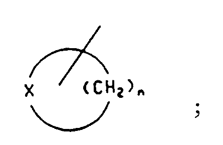 n représente 4 à 6 ; X représente O, NR⁶ ou S ; et
R⁶ représente l'hydrogène ou un groupe alkyle en C₁-C₄ ; qui consiste :
(I) à hydrolyser sélectivement un composé de formule 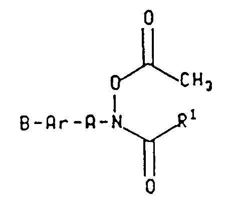 où A, B, Ar et R¹ sont tels que définis ci-dessus, avec une base choisie entre l'hydroxyde d'ammonium, l'hydroxyde de sodium, l'hydroxyde de potassium et l'hydroxyde de lithium dans un système solvant, dans des conditions comprenant une température de réaction allant de -10°C à la température ambiante ;
(II) à faire réagir un composé de formule 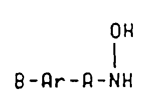 où A, B, Ar et R¹ sont tels que définis ci-dessus, avec de l'isocyanate de triméthylsilyle dans un solvant inerte vis-à-vis du milieu réactionnel, dans des conditions comprenant une température de réaction allant de la température ambiante à la température de reflux ; ou
(III) à faire réagir un composé de formule 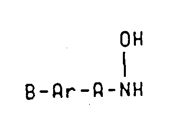 où A, B, Ar et R¹ sont tels que définis ci-dessus, avec du chlorure d'hydrogène gazeux dans un solvant inerte vis-à-vis du milieu réactionnel, dans des conditions de réaction comprenant une température de réaction allant de la température ambiante au point d'ébullition du solvant, suivi d'un traitement au phosgène.

2. Procédé selon la revendication 1, dans lequel :
lorsque l'on utilise le procédé (I), ledit système solvant est choisi parmi un ou plusieurs des solvants suivants : l'eau, le méthanol, l'éthanol, le propanol et le tétrahydrofurane ;
lorsque le procédé (II) est utilisé, ledit solvant inerte vis-à-vis du milieu réactionnel est choisi entre le tétrahydrofurane, le dioxane, le chlorure de méthylène et le benzène ; et
lorsque le procédé (III) est utilisé, ledit solvant inerte vis-à-vis du milieu réactionnel est choisi entre le benzène et le toluène.

3. Procédé selon la revendication 1 ou 2 comprenant en outre l'étape d'isolation dudit composé préparé.

4. Procédé selon l'une des revendications 1 à 3, dans lequel :
lorsque le procédé (I) est utilise, R¹ est un groupe alkyle en C₁-C₄ ; et
lorsque le procédé (II) ou (III) est utilisé, R¹ représente NR²R³.

5. Procédé selon la revendication 4, dans lequel :
lorsque le procédé (I) est utilisé, R¹ est un groupe méthyle ; et
lorsque le procédé (II) ou (III) est utilise, R¹ représente NH₂.

6. Procédé selon l'une des revendications 1 à 5, dans lequel le procédé (III) est utilisé, qui comprend en outre l'étape de traitement du mélange réactionnel avec de l'ammoniaque aqueuse.

7. Composé de formule 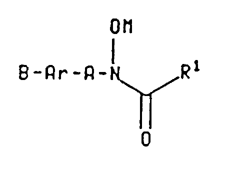 dans laquelle R¹ représente l'hydrogène, un groupe alkyle en C₁-C₄, alkényle en C₂-C₄, alkylthioalkyle, alcoxyalkyle ou NR²R³ ;
R² et R³ représentent chacun indépendamment l'hydrogène, un groupe alkyle en C₁-C₄, hydroxy, aryle ou aryle substitué par un ou plusieurs substituants choisis dans le groupe comprenant les radicaux halogéno, nitro, cyano, alkyle en C₁-C₁₂, alcoxy en C₁-C₁₂, (alkyle en C₁-C₁₂)halogéno-substitué, (alkyle en C₁-C₁₂)hydroxy-substitué, (alcoxy en C₁-C₁₂)carbonyle, aminocarbonyle, (alkyle en C₁-C₁₂)aminocarbonyle, di(alkyle en C₁-C₁₂)aminocarbonyle et (alkyle en C₁-C₁₂)sulfonyle, R² et R³ ne représentant pas simultanément hydroxy ;
M représente l'hydrogène, un cation pharmaceutiquement acceptable, un groupe aroyle ou alkoyle en C₁-C₁₂ ;
A est un groupe alkynylène ou alkynylène substitué par un ou plusieurs substituants choisis dans le groupe comprenant les radicaux alkyle en C₁-C₄, alkényle en C₂-C₄, cyano, halogéno, alcoxy en C₁-C₄, (alkyle en C₁-C₄)halogéno-substitué et (alkyle en C₁-C₄)hydroxy-substitué ;
Ar est un groupe phényle ou naphtyle ;
B représente l'hydrogène, un groupe alkyle en C₁-C₁₂, alkényle en C₂-C₁₂, (alkyle en C₁-C₁₂)halogéno-substitué, (alkyle en C₁-C₁₂) hydroxy-substitué, OR⁴, NR⁴R⁵, aryle ou aryle substitué par un ou plusieurs substituants choisis dans le groupe comprenant les radicaux halogéno, nitro, cyano, alkyle en C₁-C₆, alcoxy en C₁-C₆, (alkyle en C₁-C₆)halogéno-substitué, (alkyle en C₁-C₆)hydroxy-substitué, (alcoxy en C₁-C₆)carbonyle, aminocarbonyle, (alkyle en C₁-C₆)aminocarbonyle, di(alkyle en C₁-C₆)aminocarbonyle et (alkyle en C₁-C₆)sulfonyle ;
R⁴ et R⁵ représentent chacun indépendamment l'hydrogène, un groupe alkyle en C₁-C₁₂, un groupe alkényle en C₃-C₁₂, cycloalkyle en C₃-C₈, aryle ou 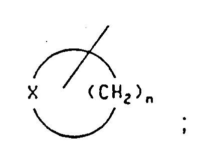 n représente 4 à 6 ; X représente O, NR⁶ ou S ; et
R⁶ représente l'hydrogène ou un groupe alkyle en C₁-C₄.

8. Composé selon la revendication 7, dans lequel :
A est un groupe alkynylène ;
R¹ est un groupe alkyle en C₁-C₄ ; et
B représente l'hydrogène, un groupe aryle ou aryloxy.

9. Composé selon la revendication 7, dans lequel :
A est un groupe alkynylène ;
R¹ est un groupe NR²R³ ; et
B représente l'hydrogène, un groupe aryle, aryloxy ou alkényloxy en C₃-C₁₂.

10. Composé selon l'une des revendications 7 à 9, dans lequel Ar est un groupe phényle.

11. Composé selon l'une des revendications 7 ou 8, dans lequel :
Ar est un groupe phényle ;
M représente l'hydrogène ; et
R¹ est un groupe méthyle.

12. Composé selon la revendication 7 ou 9, dans lequel :
Ar est un groupe phényle ;
M représente l'hydrogène ; et
R¹ est un groupe amino.

13. Composé selon l'une des revendications 7 à 12, dans lequel B est un groupe phényle ou phénoxy.

14. Composé selon l'une des revendications 7, 9 et 12, dans lequel B est un groupe allyloxy.

15. Composé selon l'une des revendications 7 à 9, dans lequel :
Ar est un groupe napthyle ; et
B représente l'hydrogène.

16. Composition pharmaceutique comprenant un composé selon l'une des revendications 7 à 15, ou un sel pharmaceutiquement acceptable d'un tel composé, et un véhicule pharmaceutiquement acceptable.

17. Composé selon l'une quelconque des revendications 7 à 15, ou sel pharmaceutiquement acceptable d'un tel composé, destiné à une utilisation comme médicament.

18. Utilisation d'un composé selon l'une quelconque des revendications 7 à 15, ou d'un sel pharmaceutiquement acceptable d'un tel composé, pour la fabrication d'un médicament pour le traitement des états allergiques ou inflammatoires ou des maladies cardio-vasculaires chez un mammifère, y compris l'être humain.

## Revendications (Revendications pour l'(les) Etat(s) contractant(s) suivant(s): ES, GR)

1. Procédé de préparation d'un composé de formule 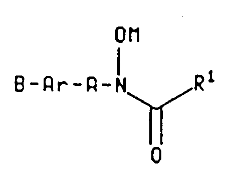 dans laquelle R¹ représente l'hydrogène, un groupe alkyle en C₁-C₄, alkényle en C₂-C₄, alkylthioalkyle, alcoxyalkyle ou NR²R³ ;
R² et R³ représentent chacun indépendamment l'hydrogè-ne, un groupe alkyle en C₁-C₄, hydroxy, aryle ou aryle substitué par un ou plusieurs substituants choisis dans le groupe comprenant les radicaux halogéno, nitro, cyano, alkyle en C₁-C₁₂, alcoxy en C₁-C₁₂, (alkyle en C₁-C₁₂)halogéno-substitué, (alkyle en C₁-C₁₂)hydroxy-substitué, (alcoxy en C₁-C₁₂)carbonyle, aminocarbonyle, (alkyle en C₁-C₁₂)aminocarbonyle, di(alkyle en C₁-C₁₂)aminocarbonyle et (alkyle en C₁-C₁₂)sulfonyle, R² et R³ ne représentant pas simultanément hydroxy ;
M représente l'hydrogène, un cation pharmaceutiquement acceptable, un groupe aroyle ou alkoyle en C₁-C₁₂ ;
A est un groupe alkynylène ou alkynylène substitué par un ou plusieurs substituants choisis dans le groupe comprenant les radicaux alkyle en C₁-C₄, alkényle en C₂-C₄, cyano, halogéno, alcoxy en C₁-C₄, (alkyle en C₁-C₄)halogéno-substitué et (alkyle en C₁-C₄)hydroxy-substitué ;
Ar est un groupe phényle ou naphtyle ;
B représente l'hydrogène, un groupe alkyle en C₁-C₁₂, alkényle en C₂-C₁₂, (alkyle en C₁-C₁₂)halogéno-substitué, (alkyle en C₁-C₁₂)hydroxy-substitué, OR⁴, NR⁴R⁵, aryle ou aryle substitué par un ou plusieurs substituants choisis dans le groupe comprenant les radicaux halogéno, nitro, cyano, alkyle en C₁-C₆, alcoxy en C₁-C₆, (alkyle en C₁-C₆)halogéno-substitué, (alkyle en C₁-C₆)hydroxy-substitué, (alcoxy en C₁-C₆)carbonyle, aminocarbonyle,(alkyle en C₁-C₆)aminocarbonyle, di(alkyle en C₁-C₆)aminocarbonyle et (alkyle en C₁-C₆)sulfonyle ;
R⁴ et R⁵ représentent chacun indépendamment l'hydrogène, un groupe alkyle en C₁-C₁₂, un groupe alkényle en C₃-C₁₂, cycloalkyle en C₃-C₈, aryle ou 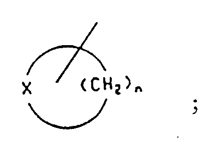 n représente 4 à 6 ; X représente O, NR⁶ ou S ; et
R⁶ représente l'hydrogène ou un groupe alkyle en C₁-C₄ ; qui consiste :
(I) à hydrolyser sélectivement un composé de formule 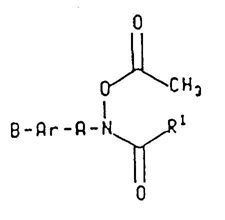 où A, B, Ar et R¹ sont tels que définis ci-dessus, avec une base choisie entre l'hydroxyde d'ammonium, l'hydroxyde de sodium, l'hydroxyde de potassium et l'hydroxyde de lithium dans un système solvant, dans des conditions comprenant une température de réaction allant de -10°C à la température ambiante ;
(II) à faire réagir un composé de formule 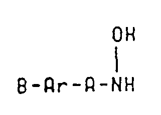 où A, B, Ar et R¹ sont tels que définis ci-dessus, avec de l'isocyanate de triméthylsilyle dans un solvant inerte vis-à-vis du milieu réactionnel, dans des conditions comprenant une température de réaction allant de la température ambiante à la température de reflux ; ou
(III) à faire réagir un composé de formule 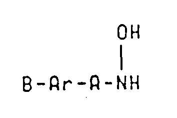 où A, B, Ar et R¹ sont tels que définis ci-dessus, avec du chlorure d'hydrogène gazeux dans un solvant inerte vis-à-vis du milieu réactionnel, dans des conditions de réaction comprenant une température de réaction allant de la température ambiante au point d'ébullition du solvant, suivi d'un traitement au phosgène.

2. Procédé selon la revendication 1, dans lequel : lorsque l'on utilise le procédé (I), ledit système solvant est choisi parmi un ou plusieurs des solvants suivants : l'eau, le méthanol, l'éthanol, le propanol et le tétrahydrofurane ;
lorsque le procédé (II) est utilisé, ledit solvant inerte vis-à-vis du milieu réactionnel est choisi entre le tétrahydrofurane, le dioxane, le chlorure de méthylène et le benzène ; et
lorsque le procédé (III) est utilisé, ledit solvant inerte vis-à-vis du milieu réactionnel est choisi entre le benzène et le toluène.

3. Procédé selon la revendication 1 ou 2 comprenant en outre l'étape d'isolation dudit composé préparé.

4. Procédé selon l'une des revendications 1 à 3, dans lequel :
lorsque le procédé (I) est utilisé, R¹ est un groupe alkyle en C₁-C₄ ; et
lorsque le procédé (II) ou (III) est utilisé, R¹ représente NR²R³.

5. Procédé selon la revendication 4, dans lequel : lorsque le procédé (I) est utilisé, R¹ est un groupe méthyle ; et
lorsque le procédé (II) ou (III) est utilisé, R¹ représente NH₂.

6. Procédé selon l'une des revendications 1 à 5, dans lequel le procédé (III) est utilisé, qui comprend en outre l'étape de traitement du mélange réactionnel avec de l'ammoniaque aqueuse.
